# EUROPEAN PATENT APPLICATION

(11) **EP 4 834 398 A1**
(43) Date of publication of application: **04.11.2026**
(21) Application number: 24909781.7
(22) Date of filing: 06.08.2024
(51) Int. Cl.: A61M 16/00

(54) **NOISE-CANCELING APPARATUS AND VENTILATION TREATMENT DEVICE**

(30) Priority: 29.12.2023 CN 202311866077
(71) Applicant: BMC Medical Co., Ltd., Beijing 100089 (CN)
(72) Inventor: LIU, Jiuzhong, Beijing 100089 (CN); ZHUANG, Zhi, Beijing 100089 (CN)
(74) Representative: Grünecker Patent- und Rechtsanwälte PartG mbB
(86) International application number: PCT/CN2024/110191
(87) International publication number: WO 2025/138827

(57) **Abstract**

The present application discloses a noise reduction apparatus and a ventilation therapy device, and belongs to the field of ventilation therapy devices. The noise reduction apparatus includes: a housing and a fan; where at least two gas chambers are provided within the housing, and the fan is disposed in one of the gas chambers; and the housing is provided with a gas inlet and a gas outlet, at least two gas passages are provided between the gas inlet and the gas outlet to enable a flow of gas from the gas inlet through the gas passages to the gas outlet, the at least two gas chambers are in communication with each other via the gas passages, and the gas passages have different path lengths.

## Description

### CROSS REFERENCE TO RELATED APPLICATION

The present application claims priority to Chinese Patent Application No. 202311866077.2, filed with the China National Intellectual Property Administration on December 29, 2023 and entitled "NOISE REDUCTION APPARATUS AND VENTILATION THERAPY DEVICE", which is incorporated herein by reference in its entirety.

### TECHNICAL FIELD

The present application relates to the technical field of ventilation therapy devices, and in particular to a noise reduction apparatus and a ventilation therapy device.

### BACKGROUND

With the development of science and technology and the improvement of medical standards, the application of ventilation therapy devices has gradually become more widespread. For example, in hospitals, a patient is required to wear a respiratory mask. The respiratory mask is connected to a ventilation therapy device, and the ventilation therapy device delivers a therapeutic gas into the respiratory mask. For another example, patients suffering from severe snoring are treated by a ventilation therapy device. Typically, a ventilation therapy device is provided with a fan that operates to cause gas to flow. However, the fan produces relatively large noise, which affects the quality of sleep of a patient and results in poor user experience for the patient using the ventilation therapy device.

### SUMMARY

Some embodiments of the present application aim to provide a noise reduction apparatus and a ventilation therapy device, in order to solve at least the problem that noise generated by a fan is relatively large, which affects the quality of sleep of a patient and results in poor user experience for the patient using the ventilation therapy device.

In a first aspect, some embodiments of the present application provide a noise reduction apparatus. The noise reduction apparatus includes a housing and a fan;
where at least two gas chambers are provided within the housing, and the fan is disposed in one of the gas chambers; and
the housing is provided with a gas inlet and a gas outlet, at least two gas passages are provided between the gas inlet and the gas outlet to enable a flow of gas from the gas inlet through the gas passages to the gas outlet, the at least two gas chambers are in communication with each other via the gas passages, and the gas passages have different path lengths.

Optionally, the path length of each of the gas passages is determined based on a frequency of noise.

Optionally, a difference between a first target gas passage and a second target gas passage is an odd multiple of half-wavelength of noise generated by the fan, such that the noise is attenuated during the flow through the first target gas passage and the second target gas passage to the gas inlet;
where the first target gas passage is at least a portion of any one of the at least two gas passages, and the second target gas passage is at least a portion of any one of the remaining gas passages;
the plurality of gas chambers are sequentially distributed at intervals within the housing; and
at least two adjacent gas chambers of the plurality of gas chambers are in communication with each other via at least two gas pipelines, and any one of the at least two gas pipelines forms at least a portion of the first target gas passage, and any one of the remaining gas pipelines forms at least a portion of the second target gas passage.

Optionally, the plurality of gas chambers are sequentially distributed at intervals within the housing;
any one of the at least two adjacent gas chambers has a plurality of inlets and one outlet; and
a space between any one of the plurality of inlets and the outlet forms a portion of the first target gas passage, and a space between any one of the remaining inlets and the outlet forms a portion of the second gas passage.

Optionally, the plurality of gas chambers are sequentially adjoined with each other within the housing; and
at least one of the plurality of gas chambers has a plurality of inlets and one outlet, a space between any one of the plurality of inlets and the outlet forms the first target gas passage, and a space between any one of the remaining inlets and the outlet forms the second gas passage.

Optionally, at least two non-adjacent gas chambers of the plurality of gas chambers are in communication with each other via at least one gas pipeline, and the at least one gas pipeline forms a portion of the first target gas passage.

Optionally, a sound-absorbing member is provided within the gas pipeline.

Optionally, a sound-absorbing member is provided on a chamber wall of the at least one gas chamber.

Optionally, the noise reduction apparatus includes an upper housing, a liner, a middle housing, and a lower housing;
where the upper housing, the middle housing, and the lower housing are sequentially connected to each other to form a cavity, the liner is located in the cavity, the lower housing and the middle housing enclose a first gas chamber, the liner and the upper housing enclose a second gas chamber, the liner and the middle housing enclose a third gas chamber, the fan is located within the third gas chamber, the first gas chamber is in communication with the second gas chamber, and the second gas chamber is in communication with the third gas chamber; and
where the first gas chamber is in communication with the second gas chamber via at least one gas passage, and the second gas chamber is in communication with the third gas chamber via at least one gas passage; and the gas inlet is located in the lower housing, and the gas outlet is located in the middle housing.

Optionally, the third gas chamber is in communication with the first gas chamber via at least one gas passage.

In a second aspect, some embodiments of the present application provide a ventilation therapy device. The ventilation therapy device includes a noise reduction apparatus of any one of the embodiments in the first aspect described above.

In some embodiments of the present application, since a plurality of gas chambers within the housing are sequentially in communication with each other and at least two gas chambers are in communication with each other via gas passages, once gas enters one of the gas chambers through the gas inlet, the gas may be delivered from this gas chamber through the gas passages to another gas chamber until the gas is delivered to the gas chamber that is in communication with the gas outlet, and finally flows out from the gas outlet. Since the fan is located in the gas chamber that is in communication with the gas outlet and the exhaust end of the fan is in communication with the gas outlet, during operation of the fan, the fan can draw gas through the gas inlet into the gas chamber that is in communication with the gas inlet, the gas flows through each gas chamber and subsequently enters the gas chamber accommodating the fan, then the gas enters an intake end of the fan, and the gas flows through the fan and flows out from the exhaust end of the fan, that is, the gas flows out from the gas outlet. Noise generated during operation of the fan may be transmitted from the gas chamber accommodating the fan to the gas chamber that is in communication with the gas inlet, that is, transmitted from the gas chamber accommodating the fan to the gas inlet. During the noise transmission process, the noise propagates through the plurality of gas chambers via the gas passages. Since the gas passages have different path lengths, noise mutually interferes when being finally transmitted to the gas inlet, such that the noise is attenuated when being transmitted to the gas inlet. In addition, during the noise transmission process, the gas chambers can cause the noise to be attenuated, and can also cause the noise to be relatively small when being transmitted to the gas outlet. That is, in some embodiments of the present application, the plurality of gas chambers are provided, at least two gas chambers are in communication with each other via the gas passages, and the fan is located in the gas chamber that is in communication with the gas outlet, such that during operation of the fan, the noise reduction apparatus can draw in gas without affecting the gas transmission, and when noise generated by the fan is transmitted through the gas passages to the gas chambers and finally to the gas inlet, the noise mutually interferes to cause the noise to be relatively small when being transmitted to the gas inlet, such that the noise heard by a patient is relatively small, which is conducive to improving the quality of sleep of the patient, thereby improving the user experience of the patient.

The above description is merely a summary of the technical solutions of the present application. To make the technical means of the present application more clearly understood and implemented according to the contents of the specification, and to make the above and other objectives, features, and advantages of the present application more obvious and comprehensible, the specific embodiments of the present application are described in detail below.

### BRIEF DESCRIPTION OF THE DRAWINGS

To describe the technical solutions in embodiments of the present application or in the prior art more clearly, the following briefly describes the accompanying drawings required for describing the embodiments or the prior art. It is clear that the accompanying drawings in the following descriptions are merely some embodiments of the present application, and those of ordinary skill in the art may still derive other drawings from these accompanying drawings without creative efforts.
FIG. 1 shows a first schematic diagram of a noise reduction apparatus according to some embodiments of the present application;
FIG. 2 shows a second schematic diagram of a noise reduction apparatus according to some embodiments of the present application;
FIG. 3 shows a third schematic diagram of a noise reduction apparatus according to some embodiments of the present application;
FIG. 4 shows a first cross-sectional view of a noise reduction apparatus according to some embodiments of the present application;
FIG. 5 shows a second cross-sectional view of a noise reduction apparatus according to some embodiments of the present application;
FIG. 6 shows a third cross-sectional view of a noise reduction apparatus according to some embodiments of the present application; and
FIG. 7 shows a fourth cross-sectional view of a noise reduction apparatus according to some embodiments of the present application.

List of reference signs:
10: Housing; 11: Upper housing; 12: Liner; 13: Middle housing; 14: Lower housing; 101: Gas inlet; 102: Gas outlet; 103: Gas chamber; 110: First gas chamber; 120: Second gas chamber; 130: Third gas chamber; 1031: Inlet; 1032: Outlet; 20: Fan; 30: Gas pipeline; 40: Sound-absorbing member.

### DETAILED DESCRIPTION OF EMBODIMENTS

A feature defined by "first" or "second" in the description and the claims of the present application may explicitly or implicitly include one or more such features. In the description of the present application, unless otherwise specified, "a plurality of" means two or more. In addition, "and/or" in the description and the claims indicates at least one of connected objects, and the symbol "/" generally indicates an "or" relationship between associated objects.

In the description of the present application, it should be understood that orientation or position relationships indicated by terms such as "center", "longitudinal", "transverse", "length", "width", "thickness", "up", "down", "front", "rear", "left", "right", "vertical", "horizontal", "top", "bottom", "inner", "outer", "clockwise", "counterclockwise", "axial", "radial", "circumferential", etc. are based on orientation or position relationships shown in the accompanying drawings and are merely for ease of description of the present application and simplification of the description, rather than indicating or implying that the apparatuses or elements referred to must have a specific orientation or be constructed and operated in a specific orientation, and therefore cannot be construed as limiting the present application.

In the description of the present application, it should be noted that unless otherwise explicitly specified and defined, terms "mounting", "connecting" and "connection" should be understood in a broad sense, for example, they may be a fixed connection, a detachable connection, or an integrated connection, may be a mechanical connection or an electrical connection; or may be a direct connection, an indirect connection by means of an intermediate medium, or internal communication between two elements. For those of ordinary skill in the art, the specific meaning of the terms mentioned above in the present application can be construed according to specific circumstances.

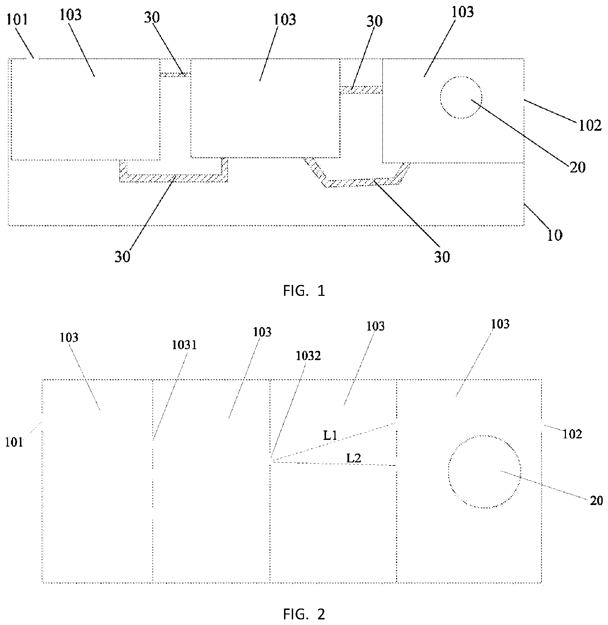

As shown in FIGS. 1 to 7, a noise reduction apparatus includes a housing 10 and a fan 20.

At least two gas chambers 103 are provided within the housing 10, and the fan is disposed in one of the gas chambers 103. The housing 10 is provided with a gas inlet 101 and a gas outlet 102, at least two gas passages are provided between the gas inlet 101 and the gas outlet 102 to enable a flow of gas from the gas inlet 101 through the gas passages to the gas outlet 102, the at least two gas chambers 103 are in communication with each other via the gas passages, and the gas passages have different path lengths.

In addition, in some embodiments of the present application, one gas chamber 103 of the plurality of gas chambers 103 is in communication with the gas inlet 101, another gas chamber 103 is in communication with the gas outlet 102, the fan 20 is located in the gas chamber 103 that is in communication with the gas outlet 102, and an exhaust end of the fan 20 is in communication with the gas outlet 102. During operation of the fan 20, noise generated by the fan 20 is transmitted from the gas chamber 103 accommodating the fan 20 to the gas inlet 101, and the gas chamber 103 attenuates the noise.

In some embodiments of the present application, since a plurality of gas chambers 103 are provided within the housing 10 and at least two gas chambers 103 are in communication with each other via gas passages, once gas enters one of the gas chambers 103 through the gas inlet 101, the gas may be delivered from this gas chamber 103 through the gas passages to another gas chamber 103 until the gas is delivered to the gas chamber 103 that is in communication with the gas outlet 102, and finally flows out from the gas outlet 102. Since the fan 20 is located in the gas chamber 103 that is in communication with the gas outlet 102 and the exhaust end of the fan 20 is in communication with the gas outlet 102, during operation of the fan 20, the fan 20 can draw gas through the gas inlet 101 into the gas chamber 103 that is in communication with the gas inlet 101, the gas flows through each gas chamber 103 and subsequently enters the gas chamber 103 accommodating the fan 20, then the gas enters an intake end of the fan 20, and the gas flows through the fan 20 and flows out from the exhaust end of the fan 20, that is, the gas flows out from the gas outlet 102. Noise generated during operation of the fan 20 may be transmitted from the gas chamber 103 accommodating the fan 20 to the gas chamber 103 that is in communication with the gas inlet 101, that is, transmitted from the gas chamber 103 accommodating the fan 20 to the gas inlet 101. During the noise transmission process, the noise propagates through the plurality of gas chambers 103 via the gas passages. Since the gas passages have different path lengths, the noise mutually interferes when being finally transmitted to the gas inlet 101, such that the noise is attenuated when being transmitted to the gas inlet 101. In addition, during the noise transmission process, the gas chambers 103 can cause the noise to be attenuated, and can also cause the noise to be relatively small when being transmitted to the outlet 1032. That is, in some embodiments of the present application, the plurality of gas chambers 103 are provided, and at least two gas chambers 103 are in communication with each other via the gas passages, such that during operation of the fan 20, the noise reduction apparatus can draw in gas without affecting the gas transmission, and when noise generated by the fan 20 is transmitted through the gas passages to the gas chambers 103 and finally to the gas inlet 101, the noise mutually interferes to cause the noise to be relatively small when being transmitted to the gas inlet 101, such that the noise heard by a patient is relatively small, which is conducive to improving the quality of sleep of the patient, thereby improving the user experience of the patient.

It should be noted that when the noise reduction apparatus according to some embodiments of the present application is applied to a ventilation therapy device, once the fan 20 operates, the fan 20 can draw gas into the gas inlet 101 of the noise reduction apparatus, such that the gas flows through the plurality of gas chambers 103, and then the gas flows out from the gas outlet 102 and flows to the patient. However, noise generated by the fan 20 propagates in a direction opposite to the flow direction of the gas, that is, noise generated by the fan 20 is transmitted from the gas chamber 103 accommodating the fan 20 to the gas inlet 101, and then the patient hears the noise. In some embodiments of the present application, by providing a plurality of gas chambers 103, the noise is attenuated by the gas chambers 103 and is thus relatively small when being transmitted to the gas inlet 101, such that the noise heard by the patient is relatively small, which is conducive to improving the quality of sleep of the patient.

In addition, in some embodiments of the present application, the path length of each gas passage is determined based on a frequency of noise. Specifically, the path length of the gas passage can be determined by determining the maximum noise based on the frequency of noise and then determining the length of the gas path based on half-wavelength of the maximum noise. Of course, it is also possible to determine the maximum noise based on the frequency of noise and then determine the length of the gas path based on half-wavelength of noise that is not the maximum noise. This is not limited in the embodiments of the present application.

In addition, in some embodiments of the present application, a difference between a first target gas passage and a second target gas passage is an odd multiple of half-wavelength of noise generated by the fan 20, such that the noise is attenuated during the flow through the first target gas passage and the second target gas passage to the gas inlet. The first target gas passage is at least a portion of any one of the at least two gas passages, and the second target gas passage is at least a portion of any one of the remaining gas passages.

Since the difference between the first target gas passage and the second target gas passage is an odd multiple of half-wavelength of noise generated by the fan 20, once the noise propagates through the first target gas passage and the second target gas passage and enters the gas chamber 103, that is, the noise finally converges in the gas chamber 103 that is in communication with the gas inlet 101, the noise will mutually interfere, thereby attenuating the noise.

In addition, in some embodiments of the present application, a plurality of gas chambers 103 are sequentially distributed at intervals within the housing 10; and at least two adjacent gas chambers 103 of the plurality of gas chambers are in communication with each other via at least two gas pipelines 30, any gas pipeline 30 of the at least two gas pipelines 30 forms at least a portion of the first target gas passage, and any gas pipeline 30 of the remaining gas pipelines forms at least a portion of the second target gas passage. That is, at least two adjacent gas chambers 103 are in communication with each other via at least two gas pipelines 30, and for the at least two gas pipelines 30, the length difference between the at least two gas pipelines 30 is an odd multiple of half-wavelength of noise generated by the fan 20, such that the noise is attenuated when propagating through the gas pipelines 30 and the gas chambers 103.

Since the length difference between at least two gas pipelines 30 in at least two adjacent gas chambers 103 is an odd multiple of half-wavelength of noise generated by the fan 20, when noise generated by the fan 20 is transmitted through the gas pipelines 30 between the two adjacent gas chambers 103 during operation of the fan 20, noise in different gas pipelines 30 mutually interferes. That is, once the noise is transmitted through different gas pipelines 30 to the gas chamber 103, the noise mutually interferes in the gas chamber 103 and is thus attenuated, such that the noise is attenuated when being transmitted to the gas outlet 102.

It should be noted that for the plurality of gas pipelines 30 between two adjacent gas chambers 103, it is possible to allow a length difference between each pair of adjacent gas pipelines 30 to be an odd multiple of half-wavelength of noise generated by the fan 20, or allow a length difference between only two of the gas pipelines 30 to be an odd multiple of half-wavelength of noise generated by the fan 20. For example, three gas pipelines 30 are provided between two adjacent gas chambers 103, namely, channel A, channel B, and channel C, and a length difference between the channel A and the channel B is an odd multiple of half-wavelength of noise generated by the fan 20. As another example, three gas pipelines 30 are provided between two adjacent gas chambers 103, namely, channel A, channel B, and channel C, a length difference between the channel A and the channel B is an odd multiple of half-wavelength of noise generated by the fan 20, and a length difference between the channel B and the channel C is an odd multiple of half-wavelength of noise generated by the fan 20.

In addition, in some embodiments of the present application, the plurality of gas chambers 103 may be divided into a plurality of groups of gas chambers, each group of gas chambers including two adjacent gas chambers 103. Two gas chambers 103 in at least one of the groups of gas chambers are in communication with each other via at least two gas pipelines 30, and two adjacent gas chambers 103 in the remaining groups of gas chambers are in communication with each other via at least one gas pipeline 30. Of course, two adjacent gas chambers 103 in each group of gas chambers may also be in communication with each other via at least two gas pipelines 30. In any case where two adjacent gas chambers 103 are in communication with each other via at least two gas pipelines 30, in the at least two gas pipelines 30, there will be gas pipelines 30 having a length difference between two gas pipelines 30 that is an odd multiple of half-wavelength of noise.

In addition, in some embodiments of the present application, a plurality of gas chambers 103 are sequentially distributed at intervals within the housing 10. In at least two adjacent gas chambers 103, any gas chamber 103 has a plurality of inlets 1031 and one outlet 1032; and a space between any inlet 1031 of the plurality of inlets 1031 and the outlet 1032 forms a portion of the first target gas passage, and a space between any one of the remaining inlets 1031 and the outlet 1032 forms a portion of the second gas passage.

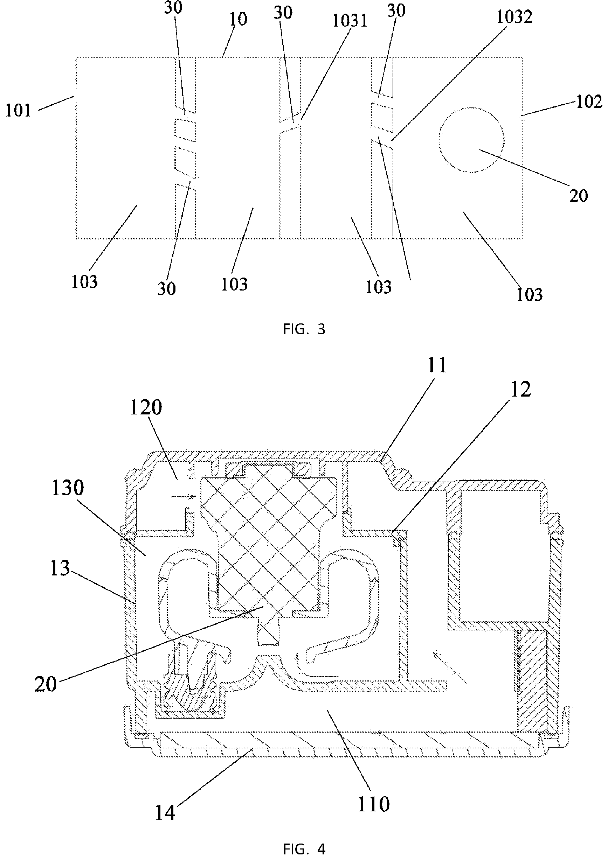

As shown in FIG. 3, a plurality of gas chambers 103 are sequentially distributed at intervals within the housing 10. The gas chambers 103 are in communication with each other via gas pipelines 30. The gas chamber 103 has a plurality of inlets 1031 and one outlet 1032, or the gas chamber 103 has a plurality of outlets 1032 and one inlet 1031. In the case where the gas chamber 103 has a plurality of inlets 1031 and one outlet 1032, a distance between one inlet 1031 of at least two inlets 1031 and the outlet 1032 is L1, a distance between another inlet 1031 and the outlet 1032 is L2, and a difference between L1 and L2 is an odd multiple of half-wavelength of noise generated by the fan 20, such that the noise is attenuated when propagating through the gas chamber 103. In the case where the gas chamber 103 has a plurality of outlets 1032 and one inlet 1031, a distance between one outlet 1032 of at least two outlets 1032 and the inlet 1031 is L3, a distance between another outlet 1032 and the inlet 1031 is L4, and a difference between L3 and L4 is an odd multiple of half-wavelength of noise generated by the fan 20, such that the noise is attenuated when propagating through the gas chamber 103. The inlet 1031 of one gas chamber 103 is in communication with the outlet of another gas chamber 1032 via a gas pipeline 30, thereby achieving communication.

In the case where the gas chamber 103 has a plurality of inlets 1031 and one outlet 1032, when two adjacent gas chambers 103 are in communication with each other via a gas pipeline 30, the gas pipeline 30 may include a main gas conduit and a plurality of branch gas conduits. The plurality of branch gas conduits are all connected to the main gas conduit, the main gas conduit is connected to the outlet 1032 of one gas chamber 103 of the two adjacent gas chambers 103, the plurality of branch gas conduits are respectively connected to the plurality of inlets 1031 of the other gas chamber 103, thereby achieving communication between the two adjacent gas chambers 103. In the case where the gas chamber 103 has a plurality of outlets 1032 and one inlet 1031, when two adjacent gas chambers 103 are in communication with each other via a gas pipeline 30, the gas pipeline 30 may include a main gas conduit and a plurality of branch gas conduits. The plurality of branch gas conduits are all connected to the main gas conduit, the main gas conduit is connected to the inlet 1031 of one gas chamber 103 of the two adjacent gas chambers 103, the plurality of branch gas conduits are respectively connected to the plurality of outlets 1032 of the other gas chamber 103, thereby achieving communication between the two adjacent gas chambers 103.

In addition, in the case where the gas chamber 103 has a plurality of inlets 1031 and one outlet 1032, by setting the difference between L1 and L2 to be an odd multiple of half-wavelength of noise generated by the fan 20, it can be ensured that after the noise from the fan 20 is transmitted into the gas chamber 103, when the noise is transmitted from the outlet 1032 of the gas chamber to the plurality of inlets 1031, the difference of a noise transmission path satisfies the odd multiple of half-wavelength of the noise, such that when the noise is transmitted from the current gas chamber 103 to a gas pipeline 30 and then through the gas pipeline 30 to another gas chamber 103, the noise mutually interferes within this gas chamber 103 and is thus attenuated, which subsequently causes the noise to be attenuated when being transmitted to the gas inlet 101.

In the case where the gas chamber 103 has a plurality of outlets 1032 and one inlet 1031, by setting the difference between L3 and L4 to be an odd multiple of half-wavelength of noise generated by the fan 20, it can be ensured that after the noise from the fan 20 is transmitted to the gas chamber 103, when the noise is transmitted from the plurality of outlets 1032 to the inlet 1031 of the gas chamber 103, the difference of the noise transmission path satisfies the odd multiple of half-wavelength of noise when the noise is transmitted from the current gas chamber 103 to a gas pipeline 30 and then through the gas pipeline 30 to another gas chamber 103, such that when the noise is transmitted from the current gas chamber 103 to another gas chamber 103, the noise mutually interferes in this gas chamber 103 and is thus attenuated, which subsequently causes the noise to be attenuated when being transmitted to the gas inlet 101.

That is, by the configuration in which, in at least two adjacent gas chambers 103, any gas chamber 103 has a plurality of inlets 1031 and one outlet 1032, a space between any one of the plurality of inlets 1031 and the outlet 1032 forms a portion of the first target gas passage, and a space between any one of the remaining inlets 1031 and the outlet 1032 forms a portion of the second gas passage, it is not only possible to cause the noise to be attenuated when being transmitted between adjacent gas chambers 103 through the gas pipeline 30, but also cause the noise to be attenuated when being transmitted in the gas chamber 103, such that the noise can be further attenuated when being transmitted to the gas inlet 101.

In addition, in some embodiments of the present application, as shown in FIG. 2, a plurality of gas chambers 103 are sequentially adjoined with each other within the housing 10. In the plurality of gas chambers 103, at least one gas chamber 103 has a plurality of inlets 1031 and one outlet 1032, a space between any one of the plurality of inlets 1031 and the outlet 1032 forms a first target gas passage, and a space between any one of the remaining inlets 1031 and the outlet 1032 forms a second gas passage. In two adjacent gas chambers 103, the outlet 1032 of one gas chamber 103 coincides with the inlet of another gas chamber 1031, that is, the outlet 1032 of one gas chamber 103 is the inlet 1031 of another gas chamber 103. In some embodiments of the present application, a plurality of gas chambers are provided.

In the case where the gas chamber 103 has a plurality of inlets 1031 and one outlet 1032, a distance between one inlet 1031 of the at least two inlets 1031 and the outlet 1032 is L1, a distance between another inlet 1031 and the outlet 1032 is L2, and a difference between L1 and L2 is an odd multiple of half-wavelength of noise generated by the fan 20, such that the noise is attenuated when propagating through the gas chamber 103. In the case where the gas chamber 103 has a plurality of outlets 1032 and one inlet 1031, a distance between one outlet 1032 of at least two outlets 1032 and the inlet 1031 is L3, a distance between another outlet 1032 and the inlet 1031 is L4, and a difference between L3 and L4 is an odd multiple of half-wavelength of noise generated by the fan 20, such that the noise is attenuated when propagating through the gas chamber 103.

In addition, in the case where the gas chamber 103 has a plurality of inlets 1031 and one outlet 1032, by setting the difference between L1 and L2 to be an odd multiple of half-wavelength of noise generated by the fan 20, it can be ensured that after the noise from the fan 20 is transmitted into the gas chamber 103, when the noise is transmitted from the outlet 1032 of the gas chamber to the plurality of inlets 1031, the difference of the noise transmission path satisfies the odd multiple of half-wavelength of the noise, such that when the noise is transmitted from the current gas chamber 103 to another gas chamber 103, the noise mutually interferes within this gas chamber 103 and is thus attenuated, which subsequently causes the noise to be attenuated when being transmitted to the gas inlet 101.

In the case where the gas chamber 103 has a plurality of outlets 1032 and one inlet 1031, by setting the difference between L3 and L4 to be an odd multiple of half-wavelength of noise generated by the fan 20, it can be ensured that after the noise from the fan 20 is transmitted into the gas chamber 103, when the noise is transmitted from the plurality of outlets 1032 of the gas chamber to the inlet 1031, the difference of the noise transmission path satisfies the odd multiple of half-wavelength of the noise, such that when the noise is transmitted from the current gas chamber 103 to another gas chamber 103, the noise mutually interferes within this gas chamber 103 and is thus attenuated, which subsequently causes the noise to be attenuated when being transmitted to the gas inlet 101.

That is, by the configuration in which, in the plurality of gas chambers 103, at least one gas chamber 103 has a plurality of inlets 1031 and one outlet 1032, a space between any inlet 1031 of the plurality of inlets 1031 and the outlet 1032 forms a first target gas passage, and a space between any inlet 1031 of the remaining inlets 1031 and the outlet 1032 forms a second gas passage, that is, the noise transmission paths are different when the noise is transmitted to the gas chambers 103, and thus the noise mutually interferes, such that the noise is attenuated when being transmitted to the gas inlet 101.

It should be noted that in some embodiments of the present application, "a plurality of" means two or more.

In addition, in some embodiments of the present application, the plurality of gas chambers 103 may be divided into a plurality of groups of gas chambers, each group of gas chambers including two adjacent gas chambers 103. In two gas chambers 103 in at least one of the groups of gas chambers, one gas chamber 103 has a plurality of inlets 1031 and one outlet 1032, and the other gas chamber 103 has one inlet 103 and a plurality of outlets 1032. In the remaining groups of gas chambers, each gas chamber 103 of the two adjacent gas chambers 103 has one inlet 1031 and one outlet 1032.

In addition, in some embodiments of the present application, when the gas chamber 103 has a plurality of inlets 1031, the number of inlets 1031 can be set according to actual needs. For example, three inlets 1031 are provided, or for another example, five inlets 1031 are provided. The specific number of inlets 1031 is not limited in some embodiments of the present application. When the gas chamber 103 has a plurality of outlets 1032, the number of outlets 1032 can be set according to actual needs. For example, three outlets 1032 are provided, or for another example, four outlets 1032 are provided. The specific number of outlets 1032 is not limited in some embodiments of the present application.

In addition, in some embodiments of the present application, at least two non-adjacent gas chambers 103 of the plurality of gas chambers 103 are in communication with each other via at least one gas pipeline 30, and the at least one gas pipeline 30 forms a portion of the first target gas passage. With such a configuration, a length difference of the gas pipeline 30 between at least two non-adjacent gas chambers 103 may be an odd multiple of half-wavelength of noise, which further causes noise to be attenuated when gas is delivered to the gas inlet 101.

In addition, in some embodiments of the present application, a sound-absorbing member 40 may be provided in the gas pipeline 30. By providing a sound-absorbing member 40 in the gas pipeline 30, the sound-absorbing member 40 may absorb part of noise when the noise is transmitted through the gas pipeline 30, which is conducive to causing the noise to be attenuated when being transmitted to the gas inlet 101.

It should be noted that the sound-absorbing member 40 may be sound-absorbing cotton that can be attached within the gas pipeline 30 and cover the passage wall of the gas pipeline 30. Of course, the sound-absorbing member 40 may also be of other types, for example, the sound-absorbing member 40 is formed of silicone. The specific type of the sound-absorbing member 40 is not limited in some embodiments of the present application.

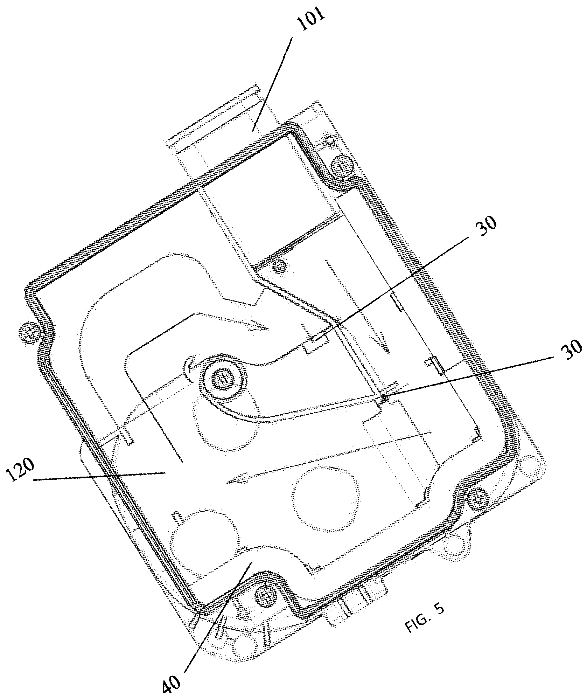

In addition, in some embodiments of the present application, as shown in FIG. 5, at least one gas chamber 103 is provided with a sound-absorbing member 40 on its chamber wall. By providing a sound-absorbing member 40 in the gas chamber 103, the sound-absorbing member 40 may absorb part of noise when the noise is transmitted through the gas chamber 103, which is conducive to causing the noise to be attenuated when being transmitted to the gas inlet 101.

It should be noted that the sound-absorbing member 40 may be provided in one of the gas chambers 103, or the sound-absorbing member 40 may be provided in each gas chamber 103, or the sound-absorbing member 40 may be provided in each of some gas chambers 103 of the plurality of gas chambers 103. This is not limited in some embodiments of the present application.

Of course, in some embodiments of the present application, none of the gas chambers may be provided with the sound-absorbing member 40.

In addition, in some embodiments of the present application, as shown in FIG. 4, the noise reduction apparatus includes an upper housing 11, a liner 12, a middle housing 13, and a lower housing 14. The upper housing 11, the middle housing 13, and the lower housing 14 are sequentially connected to each other to form a cavity, the liner 12 is located in the cavity, the lower housing 14 and the middle housing 13 enclose a first gas chamber 110, the liner 12 and the upper housing 11 enclose a second gas chamber 120, the liner 12 and the middle housing 13 enclose a third gas chamber 130, the fan 20 is located in the third gas chamber 130, the first gas chamber 110 is in communication with the second gas chamber 120, and the second gas chamber 120 is in communication with the third gas chamber 130. The first gas chamber 110 and the second gas chamber 120 are in communication with each other via at least one gas passage, and the second gas chamber 110 and the third gas chamber 130 are in communication with each other via at least one gas passage. The gas inlet 101 is located in the lower housing 14, and the gas outlet 102 is located in the middle housing 13.

Since the gas inlet 101 is located in the lower housing 14, the gas outlet 102 is located in the middle housing 13, and the fan 20 is located in the third gas chamber 130, during operation of the fan 20, the gas may enter the first gas chamber 110 from the gas inlet 101, then flow from the first gas chamber 110 into the second gas chamber 120, and finally flow from the second gas chamber 120 into the third gas chamber 130, and the gas may then flow into the fan 20, and then flow out of the noise reduction apparatus from an exhaust end of the fan 20, that is, flow out of the noise reduction apparatus from the gas outlet 102. Noise generated by the fan 20 is transmitted from the third gas chamber 130 to the second gas chamber 120, and then transmitted from the second gas chamber 120 to the first gas chamber 110. During the noise transmission process, the noise is attenuated by the gas chamber 103, such that the noise is attenuated when being transmitted to the gas inlet 101.

It should be noted that two gas pipelines 30 may be provided between the first gas chamber 110 and the second gas chamber 120, a length difference between the two gas pipelines 30 is an odd multiple of half-wavelength of the noise, such that when the noise is transmitted from the second gas to the first gas chamber 110, the noise converges in the first gas chamber 110 and mutually interferes in the first gas chamber 110, thereby attenuating the noise. Of course, three gas pipelines 30 may also be provided between the first gas chamber 110 and the second gas chamber 120. The number of gas pipelines 30 between the first gas chamber 110 and the second gas chamber 120 is not limited in some embodiments of the present application.

In addition, in some embodiments of the present application, when a plurality of gas chambers 103 are provided within the housing 10, two adjacent gas chambers 103 are in communication with each other. Non-adjacent gas chambers 103 may also be in communication with each other.

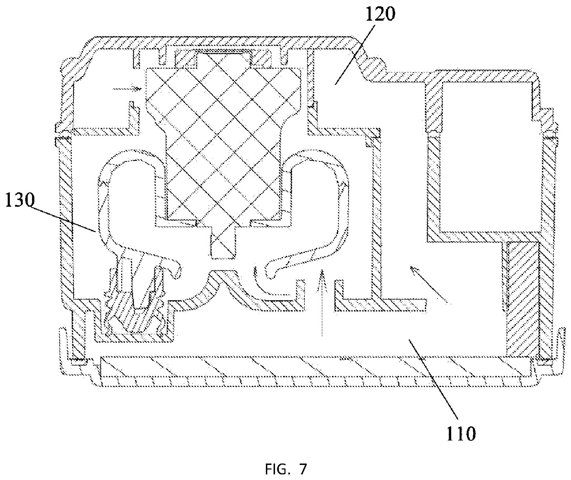

In addition, in some embodiments of the present application, as shown in FIG. 7, the third gas chamber 130 is in communication with the first gas chamber 110 via at least one gas passage. The third gas chamber 130 is not adjacent to the first gas chamber 110, and the third gas chamber 130 is in communication with the first gas chamber 110. Specifically, a gas delivery path, that is, a gas passage, may be provided between the third gas chamber 130 and the first gas chamber 110, such that noise from the third gas chamber 130 directly propagates to the first gas chamber 110, and in this case the noise propagating from the third gas chamber 130 and the second gas chamber 120 to the first gas chamber 110 and the noise directly propagating from the third gas chamber 130 to the first gas chamber 110 have different noise propagation paths and different propagation distances, such that the noise that converges in the first gas chamber 110 mutually interferes, thereby attenuating the noise.

It should be noted that at least one gas passage may be provided between the third gas chamber 130 and the first gas chamber 110. For example, two gas passages are provided between the third gas chamber 130 and the first gas chamber 110, and a length difference between the two gas passages is an odd multiple of half-wavelength of noise.

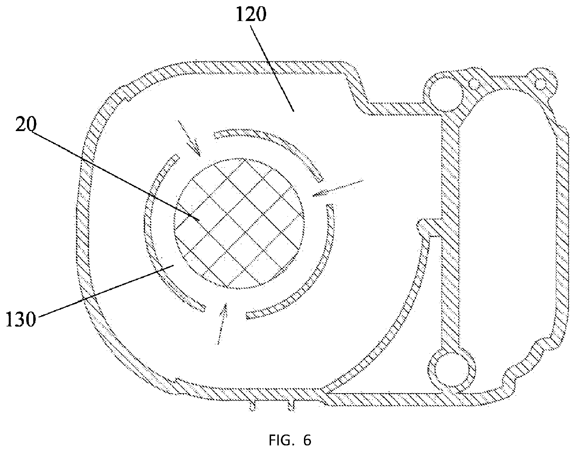

In addition, in some embodiments of the present application, as shown in FIG. 6, the second gas chamber 120 may be annular, and in this case the center of the annulus is a portion of the third gas chamber 130, and a motor of the fan 20 may be located at this position. In addition, the second gas chamber 120 is in communication with the third gas chamber 130 via a plurality of openings.

As shown in FIG. 6, three gas pipelines 30 are provided for the connection between the second gas chamber 120 and the third gas chamber 130, and when noise propagates from the third gas chamber 130 to the second gas chamber 120 and converges in the second gas chamber 120, owing to different path lengths, the noise mutually interferes, thereby attenuating the noise. In addition, since the motor of the fan 20 has a high speed and generates a lot of heat, the gas flowing from the second gas chamber 120 into the third gas chamber 130 is equivalent to blowing towards the motor from different directions, which can also dissipate heat for the motor.

Since a plurality of gas chambers 103 are provided within the housing 10 and at least two gas chambers 103 are in communication with each other via gas passages, once gas enters one of the gas chambers 103 from the gas inlet 101, the gas may be delivered from this gas chamber 103 through the gas passages to another gas chamber 103 until the gas is delivered to the gas chamber 103 that is in communication with the gas outlet 102, and finally flows out from the gas outlet 102. Since the fan 20 is located in the gas chamber 103 that is in communication with the gas outlet 102 and the exhaust end of the fan 20 is in communication with the gas outlet 102, during operation of the fan 20, the fan 20 can draw gas through the gas inlet 101 into the gas chamber 103 that is in communication with the gas inlet 101, the gas flows through each gas chamber 103 and subsequently enters the gas chamber 103 accommodating the fan 20, then the gas enters an intake end of the fan 20, and the gas flows through the fan 20 and flows out from the exhaust end of the fan 20, that is, the gas flows out from the gas outlet 102. Noise generated during operation of the fan 20 may be transmitted from the gas chamber 103 accommodating the fan 20 to the gas chamber 103 that is in communication with the gas inlet 101, that is, transmitted from the gas chamber 103 accommodating the fan 20 to the gas inlet 101. During the noise transmission process, the noise propagates through the plurality of gas chambers 103 via the gas passages. Since the gas passages have different path lengths, the noise mutually interferes when being finally transmitted to the gas inlet 101, such that the noise is attenuated when being transmitted to the gas inlet 101. In addition, during the noise transmission process, the gas chambers 103 can cause the noise to be attenuated, and can also cause the noise to be relatively small when being transmitted to the outlet 1032. That is, in some embodiments of the present application, the plurality of gas chambers 103 are provided, and at least two gas chambers 103 are in communication with each other via the gas passages, such that during operation of the fan 20, the noise reduction apparatus can draw in gas without affecting the gas transmission, and when noise generated by the fan 20 is transmitted through the gas passages to the gas chambers 103 and finally to the gas inlet 101, the noise mutually interferes to cause the noise to be relatively small when being transmitted to the gas inlet 101, such that the noise heard by a patient is relatively small, which is conducive to improving the quality of sleep of the patient, thereby improving the user experience of the patient.

Some embodiments of the present application provide a ventilation therapy device. The ventilation therapy device includes a noise reduction apparatus in any one of the embodiments described above.

In the description of the specification, the description with reference to terms such as "an embodiment", "some embodiments", "a schematic embodiment", "an example", "a specific example", or "some examples" means that specific features, structures, materials, or characteristics described in combination with the embodiments or examples are included in at least one embodiment or example of the present application. In this specification, the schematic expressions of the above terms do not necessarily refer to the same embodiments or examples. Furthermore, the specific features, structures, materials, or characteristics described may be combined in a suitable manner in any one or more embodiments or examples.

Although some embodiments of the present application have been shown and described, those of ordinary skill in the art can understand that various changes, modifications, substitutions, and variations can be made to these embodiments without departing from the principles and spirit of the present application. The scope of the present application is defined by the claims and equivalents thereof.

## Claims

1. A noise reduction apparatus, comprising a housing and a fan;
wherein at least two gas chambers are provided within the housing, and the fan is disposed in one of the gas chambers; and
the housing is provided with a gas inlet and a gas outlet, at least two gas passages are provided between the gas inlet and the gas outlet to enable a flow of gas from the gas inlet through the gas passages to the gas outlet, the at least two gas chambers are in communication with each other via the gas passages, and the gas passages have different path lengths.

2. The noise reduction apparatus according to claim 1, wherein the path length of each of the gas passages is determined based on a frequency of noise.

3. The noise reduction apparatus according to claim 2, wherein a difference between a first target gas passage and a second target gas passage is an odd multiple of half-wavelength of noise generated by the fan, such that the noise is attenuated during the flow through the first target gas passage and the second target gas passage to the gas inlet;
wherein the first target gas passage is at least a portion of any one of the at least two gas passages, and the second target gas passage is at least a portion of any one of the remaining gas passages.

4. The noise reduction apparatus according to claim 3, wherein the plurality of gas chambers are sequentially distributed at intervals within the housing; and
at least two adjacent gas chambers of the plurality of gas chambers are in communication with each other via at least two gas pipelines, any one of the at least two gas pipelines forms at least a portion of the first target gas passage, and any one of the remaining gas pipelines forms at least a portion of the second target gas passage.

5. The noise reduction apparatus according to claim 4, wherein the plurality of gas chambers are sequentially distributed at intervals within the housing;
any one of the at least two adjacent gas chambers has a plurality of inlets and one outlet; and
a space between any one of the plurality of inlets and the outlet forms a portion of the first target gas passage, and a space between any one of the remaining inlets and the outlet forms a portion of the second gas passage.

6. The noise reduction apparatus according to claim 3, wherein the plurality of gas chambers are sequentially adjoined with each other within the housing; and
at least one of the plurality of gas chambers has a plurality of inlets and one outlet, a space between any one of the plurality of inlets and the outlet forms the first target gas passage, and a space between any one of the remaining inlets and the outlet forms the second gas passage.

7. The noise reduction apparatus according to claim 4-6, wherein at least two non-adjacent gas chambers of the plurality of gas chambers are in communication with each other via at least one gas pipeline, and the at least one gas pipeline forms a portion of the first target gas passage.

8. The noise reduction apparatus according to claim 4, wherein a sound-absorbing member is provided within the gas pipeline.

9. The noise reduction apparatus according to claim 1, wherein a sound-absorbing member is provided on a chamber wall of the at least one gas chamber.

10. The noise reduction apparatus according to claim 1, comprising an upper housing, a liner, a middle housing, and a lower housing;
wherein the upper housing, the middle housing, and the lower housing are sequentially connected to each other to form a cavity, the liner is located in the cavity, the lower housing and the middle housing enclose a first gas chamber, the liner and the upper housing enclose a second gas chamber, the liner and the middle housing enclose a third gas chamber, the fan is located within the third gas chamber, the first gas chamber is in communication with the second gas chamber, and the second gas chamber is in communication with the third gas chamber; and
wherein the first gas chamber is in communication with the second gas chamber via at least one gas passage, and the second gas chamber is in communication with the third gas chamber via at least one gas passage; and the gas inlet is located in the lower housing, and the gas outlet is located in the middle housing.

11. The noise reduction apparatus according to claim 10, wherein the third gas chamber is in communication with the first gas chamber via at least one gas passage.

12. A ventilation therapy device, comprising a noise reduction apparatus according to claim 1-11.
